# EUROPEAN PATENT SPECIFICATION

(11) **EP 1 293 514 B1**
(45) Date of publication and mention of the grant of the patent: **29.11.2006**
(21) Application number: 01122104.1
(22) Date of filing: 14.09.2001
(51) Int. Cl.: C07K 16/00, C07K 16/28, C07K 16/30, C07K 19/00, C12N 15/13, C12N 15/62, C12N 15/63, C12N 5/10, A61P 31/00

(54) **Multimeric single chain tandem Fv-antibodies**
Multimerische, einzelkettige, Tandem-Fv-Antikörper
Multimères d'anticorps Fv monocaténaires en tandem

(43) Date of publication of application: 19.03.2003
(73) Proprietor: Affimed Therapeutics AG, 69120 Heidelberg (DE)
(72) Inventor: Le Gall, Fabrice, 68535 Erdingen-Neckarhausen (DE); Kipriyanov, Sergey, 69126 Heidelberg (DE); Reusch, Uwe, 67487 Maikammer (DE); Moldenhauer, Gerhard, 69120 Heidelberg (DE); Little, Melvyn, 69151 Neckargemünd (DE)
(74) Representative: Schüssler, Andrea

(56) References cited:
- WO-A-99/57150
- DE-A- 19 816 141
- DE-A- 19 930 433
- US-A- 6 025 165
- KIPRIYANOV ET AL: "Bispecific tandem diabody for tumour therapy with improved antigen binding and pharmacokinetics" J.MOL.BIOL., vol. 293, pages 41-56, XP002185480
- VÖLKEL: "Optimized linker sequences for the expression of monomeric and dimeric bispecific single-chain antibodies" PROTEIN ENGINEERING , vol. 14, no. 10, October 2001 (2001-10), pages 815-823, XP002185481

## Description

The present invention relates to multimeric Fv-antibody constructs, expression vectors encoding said Fv-antibody constructs and diagnostic as well as therapeutic uses of said multimeric Fv-antibody constructs.

Natural antibodies are themselves dimers and thus bivalent. If two hybridoma cells producing different antibodies are artificially fused, some of the antibodies produced by the hybrid hybridoma are composed of two monomers with different specificities. Such bispecific antibodies can also be produced by chemically conjugating two antibodies. Natural antibodies and their bispecific derivatives are relatively large and expensive to produce. The constant domains of mouse antibodies are also a major cause of the human anti-mouse (HAMA) response, which prevents their extensive use as therapeutic agents. They can also give rise to unwanted effects due to their binding of Fc-receptors. For these reasons, molecular immunologists have been concentrating on the production of the much smaller Fab- and Fv-fragments in microorganisms. These smaller fragments are not only much easier to produce, they are also less immunogenic, have no effector functions and because of their relatively small size they are better able to penetrate tissues and tumors. In the case of the Fab-fragments, the constant domains adjacent to the variable domains play a major role in stabilizing the heavy and light chain dimer.

The Fv-fragment is much less stable and a peptide linker was therefore introduced between the heavy and light chain variable domains to increase stability. This construct is known as a single chain Fv(scFv)-fragment. A disulfide bond is sometimes introduced between the two domains for extra stability. Thus far, tetravalent scFv-based antibodies were produced by fusion to extra polymerizing domains such as the streptavidin monomer that forms tetramers and to amphipathic alpha helices. However, these extra domains can increase the immunogenicity of the tetravalent molecule.

Bivalent and bispecific antibodies can be constructed using only antibody variable domains. A fairly efficient and relatively simple method is to make the linker sequence between the V_{H} and V_{L} domains so short that they cannot fold over and bind one another. Reduction of the linker length to 3-12 residues prevents the monomeric configuration of the scFv molecule and favors intermolecular V_{H}-V_{L} pairings with formation of a 60 kDa non-covalent scFv dimer "diabody" (Holliger *et al*., 1993, *Proc. Natl. Acad. Sci. USA* 90, 6444-6448). The diabody format can also be used for generation of recombinant bispecific antibodies, which are obtained by the noncovalent association of two single-chain fusion products, consisting of the V_{H} domain from one antibody connected by a short linker to the V_{L} domain of another antibody. Reducing the linker length still further below three residues can result in the formation of trimers ("triabody", ∼ 90 kDa) or tetramers ("tetrabody", - 120 kDa) (Le Gall *et al*., 1999, *FEBS Letters* 453, 164-168). However, the small size of bispecific diabodies (50-60 kDa) leads to their rapid clearance from the blood stream through the kidneys, thus requiring the application of relatively high doses for therapy. Moreover, bispecific diabodies have only one binding domain for each specificity. However, bivalent binding is an important means of increasing the functional affinity and possibly the selectivity for particular cell types carrying densely clustered antigens.

Kipriyanov et al. (J.Mol.Biol., vol. 293, pages 41-56) describe a multimeric Fv antibody consisting of four variable domains. The four domains are bound by three linkers. The linkers are either short (12 amino acid residues) which leads to a tandem diabody formation (all antigen binding sites are formed by the respective VL or VH domains of two different chains with four domains each) or the central linker is "long" (27 amino acid residues) and the other two linkers are "short" (12 amino acid residues) - which leads to the formation of single chain diabody (all antigens are formed by the respective VL and VH domains of the same chain). The same is also disclosed in WO 99/57510. A similar construct having a central "long" linker and 2 "short" external linkers is disclosed in DE 198 16 141. A tandem single chain construct consisting of four variable domains all linked by one single amino acid is described in DE 199 30 433. On the other hand, US 6 025 165 describes a single chain antibody consisting of four variable domains linked by a central linker having one amino acid and two external linkers having 14 amino acids, whereas two antigen-binding sites are formed within the same chain.

Thus, the technical problem underlying the present invention is to provide new multimeric antibodies which overcome the disadvantages of the Fv-antibodies of the prior art and to provide a general way to form a multimeric Fv-molecule with at least four binding domains which is monospecific or multispecific.

The solution to said technical problem is achieved by providing the embodiments characterized in the claims.

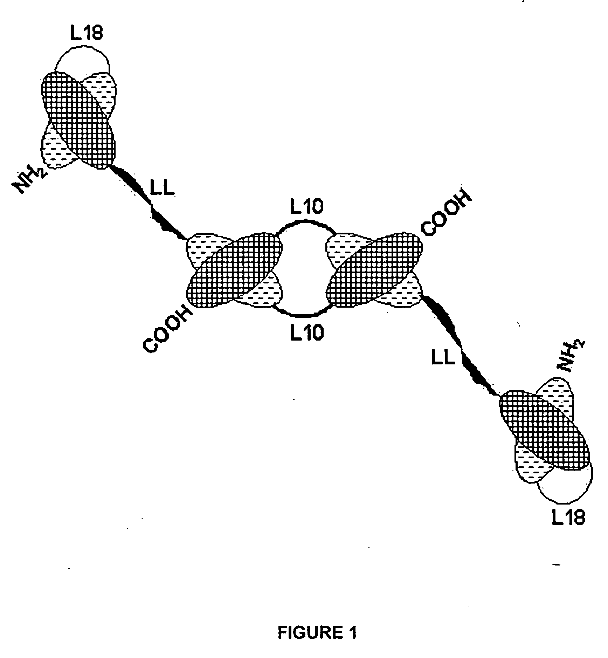

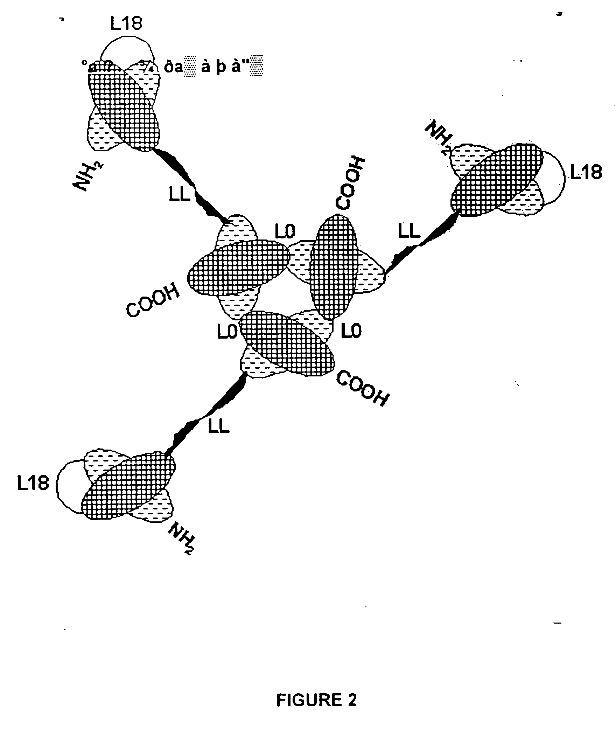

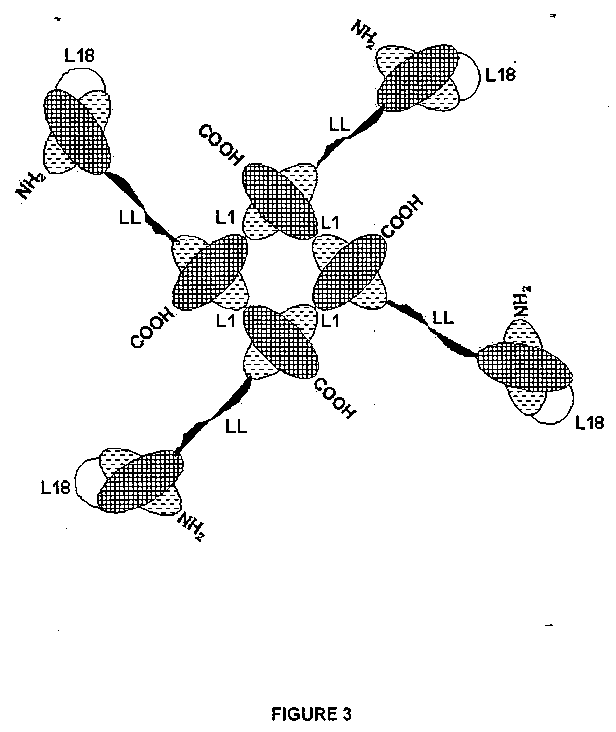

The present invention is based on the observation that scFv-dimers, -trimers and -tetramers can be used as multimerisation motifs for construction of multimeric Fv-molecules and that for the production of the multimeric Fv molecules, the peptide linker introduced between the first and second variable domain of a monomer and the second and third domain of a monomer must be long enough to permit the molecule to fold over itself, i.e. the peptide linker should have a length of at least 12 amino acids, preferably of at least 15 amino acids. Thus, the present invention provides a general way to form a multimeric Fv molecule with at least four binding domains which is monospecific or multispecific. Each monomer of the Fv molecule of the present invention is characterized by a V_{H}/V_{L} antigen-binding unit and two antibody variable domains that form V_{H}/V_{L} antigen-binding units after binding non-covalently to the variable domains of other monomers (multimerization motif). Dimers, trimers or tetramers are formed depending on the variable domains and the length of the peptide linkers between the variable domains that comprise the multimerization motif (see Figure 1, 2 and 3).

The multimeric Fv-antibodies of the present invention are expected to be very stable and have a higher binding_capacity. They should also be particularly useful for therapeutic purposes, since the dimeric diabodies used so far are small and remove fairly quickly from the blood stream through the kidneys. Moreover, the single chain format of the multimeric Fv-antibodies of the present invention allows them to be made in eukaryotic organisms and not only in bacteria.

Accordingly, the present invention relates to a multimeric Fv-antibody, characterized by the following features:
(a) two neighboring variable domains that form an antigen-binding V_{H}-VL or V_{L}-V_{H} scFv unit; these two variable domains are linked by a peptide linker of at least 12 amino acid residues;and
(b) two other neighboring variable domains that are non-covalently bound to two variable domains of another monomer of the Fv-antibody resulting in the formation of two additional antigen binding sites to form the multimerization motif; these two variable domains of each monomer are linked by a peptide linker consisting of a maximun of 10 amino acid residues.

A further preferred feature is that the antigen-binding V_{H}-V_{L} or V_{L}-V_{H} scFv unit formed by the two neighboring domains of one monomer is linked to the other variable domains of the multimerization motif by a peptide linker of at least 15 amino acid residues, preferably of at least 20 amino acid residues.

The term "Fv-antibody" relates to an antibody containing variable domains but not constant domains.

The term "peptide linker" relates to any peptide capable of connecting two variable domains with its length depending on the kinds of variable domains to be connected. The peptide linker might contain any amino acid residue with the amino acid residues glycine, serine and proline being preferred for the peptide linker linking the second and third variable domain.

A multimeric Fv-antibody of the present invention can be prepared according to standard methods. Preferably, said Fv-antibody is prepared by ligating DNA sequences encoding the peptide linkers with the DNA sequences encoding the variable domains such that the peptide linkers connect the variable domains resulting in the formation of a DNA sequence encoding a monomer of the multimeric Fv-antibody and expressing DNA sequences encoding the various monomers in a suitable expression system as described in the Examples, below.

The Fv-antibodies of the present invention can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of a variable domain or peptide linker are well known to the person skilled in the art; see, e.g., Sambrook, Molecular Cloning A Laboratory Manual, Cold Spring Harbor Laboratory (1989) N.Y.

The Fv-antibodies of the present invention can comprise at least one further protein domain, said protein domain being linked by covalent or non-covalent bonds. The linkage can be based on genetic fusion according to the methods known in the art and described above or can be performed by, e.g., chemical cross-linking as described in, e.g., WO 94/04686. The additional domain present in the fusion protein comprising the Fv-antibody employed in accordance with the invention may preferably be linked by a flexible linker, advantageously a peptide linker, wherein said peptide linker comprises plural, hydrophilic, peptide-bonded amino acids of a length sufficient to span the distance between the C-terminal end of said further protein domain and the N-terminal end of the Fv-antibody or vice versa. The above described fusion protein may further comprise a cleavable linker or cleavage site for proteinases. The fusion protein may also comprise a tag, like a histidine-tag, e.g. (His)₆.

In a preferred embodiment of the present invention, the monomers of the multimeric Fv-antibody comprise four variable domains and the third and fourth variable domains of the monomers are linked by a peptide linker of 10 or less amino acid residues, preferably less than five amino acid residues. In an even more preferred embodiment, the third and fourth domain are linked directly without intervening amino acid residues.

In another preferred embodiment of the multimeric Fv-antibody of the present invention, the second and third variable domains of the monomers of the antibody are linked by a peptide linker of at least 12 amino acid residues, preferably of at least 15 amino acid residues. Preferably, the maximum number of amino acid residues is 30. In an even more preferred embodiment, said peptide linker has the amino acid sequence (Gly₄Ser)₄.

In another preferred embodiment of the multimeric Fv-antibody of the present invention, the third and/or fourth variable domain are shortened by at least one amino acid residue at their N- and/or C-terminus. In some circumstances this truncated form give a better stability of the molecule as described in German patent application 100 63 048.0.

In a particularly preferred embodiment of the multimeric Fv-antibody of the present invention, the order of domains of a monomer is V_{H}-V_{L}.

In some cases it might be desirable to strengthen the association of two variable domains. Accordingly, in a further preferred embodiment of the multimeric Fv-antibody of the present invention, the binding of at least one pair of variable domains is strengthened by at least one intermolecular disulfide bridge. This can be achieved by modifying the DNA sequences encoding the variable domains accordingly, i.e. by introducing cysteine codons. The two most promising sites for introducing disulfide bridges appeared to be V_{H}44-V_{L}100 connecting framework 2 of the heavy chain with framework 4 of the light chain and V_{H}105-V_{L}43 that links framework 4 of the heavy chain with framework 2 of the light chain.

In a further preferred embodiment of the present invention, the multimeric Fv-antibody is a tetravalent dimer, hexavalent trimer or octavalent tetramer. The formation of such forms is preferably determined by particular V_{H} and V_{L} domains comprising the multimerization motif and by the length of the linker.

In another preferred embodiment of the present invention, the multimeric Fv-antibody is a bispecific, trispecific, tetraspecific antibody.

Multimerization of the monomeric subunits can be facilitated by the presence of a dimerization motif at the C-terminus of the fourth variable domain, which is, preferably, a (poly)peptide directly linked via a peptide bond. Examples of such dimerization motifs are known to the person skilled in the art and include streptavidin, amphipatic alpha helixes. Accordingly, in a further preferred embodiment, a dimerization motive is fused to the last domain of at least two monomers of the multimeric Fv-antibody of the present invention.

For particular therapeutic applications at least one monomer of the multimeric antibody of the invention can be linked non-covalently or covalently to a biologically active substance (e.g. cytokines or growth hormones), a chemical agent (e.g. doxorubicin, cyclosporin), a peptide (e.g. α-Amanitin), a protein (e.g. granzyme A and B).

In an even more preferred embodiment, the multimeric Fv-antibody of the present invention is (I) a monospecific antibody capable of specifically binding the CD19 antigen of B-lymphocytes or the carcinoma embryonic antigen (CEA); or (II) a bispecific antibody capable of specifically binding (a) CD19 and the CD3 complex of the T-cell receptor, (b) CD19 and the CD5 complex of the T-cell receptor, (c) CD19 and the CD28 antigen on T-lymphocytes, (d) CD19 and CD16 on natural killer cells, macrophages and activated monocytes, (e) CEA and CD3, (f) CEA and CD28, or (g) CEA and CD16. The nucleotide sequences of the variable domains have already been obtained and described in the case of the antibody anti-CD19 (Kipriyanov et al., 1996, J. Immunol. Methods 200, 51-62), anti-CD3 (Kipriyanov et al., 1997, Protein Engeng. 10, 445-453), anti-CD28 (Takemura et al., 2000, FEBS Lett. 476, 266-271), anti-CD16 (German Patent Application DE 199 37 264 A1), anti-CEA (Griffiths et al., 1993, EMBO J. 12, 725-734) and anti-CD5 (Better et al., 1993, Proc. Natl. Acad. Sci. U.S.A. 90, 457-461).

Another object of the present invention is a process for the preparation of a multimeric Fv-antibody according to the present invention, wherein (a) DNA sequences encoding the peptid linkers are ligated with the DNA sequences encoding the variable domains such that the peptide linkers connect the variable domains resulting in the formation of a DNA sequence encoding a monomer of the multimeric Fv-antibody and (b) the DNA sequences encoding the various monomers are expressed in a suitable expression system. The various steps of this process can be carried according to standard methods, e.g. methods described in Sambrook et al., or described in the Examples, below.

The present invention also relates to DNA sequences encoding the multimeric Fv-antibody of the present invention and vectors, preferably expression vectors containing said DNA sequences.

A variety of expression vector/host systems may be utilized to contain and express sequences encoding the multimeric Fv-antibody. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus, CaMV; tobacco mosaic virus, TMV) or with bacterial expression vectors (e.g., Ti or pBR322 plasmids); or animal cell systems. The invention is not limited by the host cell employed.

The "control elements" or "regulatory sequences" are those non-translated regions of the vector-enhancers, promoters, 5' and 3' untranslated regions-which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the Bluescript.RTM. phagemid (Stratagene, LaJolla, Calif.) or pSport1.TM. plasmid (Gibco BRL) and the like may be used. The baculovirus polyhedrin promoter may be used in insect cells. Promoters or enhancers derived from the genomes of plant cells (e.g., heat shock, RUBISCO; and storage protein genes) or from plant viruses (e.g., viral promoters or leader sequences) may be cloned into the vector. In mammalian cell systems, promoters from mammalian genes or from mammalian viruses are preferable. If it is necessary to generate a cell line that contains multiple copies of the sequence encoding the multimeric Fv-antibody, vectors based on SV40 or EBV may be used with an appropriate selectable marker.

In bacterial systems, a number of expression vectors may be selected depending upon the use intended for the multimeric Fv-antibody. Vectors suitable for use in the present invention include, but are not limited to the pSKK expression vector for expression in bacteria.

In the yeast, Saccharomyces cerevisiae, a number of vectors containing constitutive or inducible promoters such as alpha factor, alcohol oxidase, and PGH may be used. For reviews, see Grant et al. (1987) Methods Enzymol. 153:516-544.

In cases where plant expression vectors are used, the expression of sequences encoding the multimeric Fv-antibody may be driven by any of a number of promoters. For example, viral promoters such as the 35S and 19S promoters of CaMV may be used alone or in combination with the omega leader sequence from TMV (Takamatsu, N. (1987) EMBO J. 6:307-311). Alternatively, plant promoters such as the small subunit of RUBISCO or heat shock promoters may be used (Coruzzi, G. et al. (1984) EMBO J. 3:1671-1680; Broglie, R. et al. (1984) Science 224:838-843; and Winter, J. et al. (1991) Results Probl. Cell Differ. 17:85-105). These constructs can be introduced into plant cells by direct DNA transformation or pathogen-mediated transfection. Such techniques are described in a number of generally available reviews (see, for example, Hobbs, S. or Murry, L. E. in McGraw Hill Yearbook of Science and Technology (1992) McGraw Hill, New York, N.Y.; pp. 191-196.

An insect system may also be used to express the multimeric Fv-antibody. For example, in one such system, Autographa californica nuclear polyhedrosis virus (AcNPV) is used as a vector to express foreign genes in Spodoptera frugiperda cells or in Trichoplusia larvae. The sequences encoding the multimeric Fv-antibody may be cloned into a non-essential region of the virus, such as the polyhedrin gene, and placed under control of the polyhedrin promoter. Successful insertion of the multimeric Fv-antibody will render the polyhedrin gene inactive and produce recombinant virus lacking coat protein. The recombinant viruses may then be used to infect, for example, S. frugiperda cells or Trichoplusia larvae in which APOP may be expressed (Engelhard, E. K. et al. (1994) Proc. Nat. Acad. Sci. 91:3224-3227).

In mammalian host cells, a number of viral-based expression systems may be utilized. In cases where an adenovirus is used as an expression vector, sequences encoding the multimeric Fv-antibody may be ligated into an adenovirus transcription/translation complex consisting of the late promoter and tripartite leader sequence. Insertion in a non-essential E1 or E3 region of the viral genome may be used to obtain a viable virus which is capable of expressing the multimeric Fv-antibody in infected host cells (Logan, J. and Shenk, T. (1984) Proc. Natl. Acad. Sci. 81:3655-3659). In addition, transcription enhancers, such as the Rous sarcoma virus (RSV) enhancer, may be used to increase expression in mammalian host cells.

Human artificial chromosomes (HACs) may also be employed to deliver larger fragments of DNA than can be contained and expressed in a plasmid. HACs of 6 to 10M are constructed and delivered via conventional delivery methods (liposomes, polycationic amino polymers, or vesicles) for therapeutic purposes.

Specific initiation signals may also be used to achieve more efficient translation of sequences encoding the multimeric Fv-antibody. Such signals include the ATG initiation codon and adjacent sequences. In cases where sequences encoding the multimeric Fv-antibody, its initiation codon, and upstream sequences are inserted into the appropriate expression vector, no additional transcriptional or translational control signals may be needed. However, in case where only coding sequence is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf, D. et al. (1994) Results Probl. Cell Differ. 20:125-162).

In addition, a host cell strain may be chosen for its ability to modulate the expression of the inserted sequences or to process the expressed protein in the desired fashion. Post-translational processing which cleaves a "prepro" form of the protein may also be used to facilitate correct insertion, folding and/or function. Different host cells which have specific cellular machinery and characteristic mechanisms for post-translational activities (e.g., CHO, HeLa, MDCK, HEK293, and W138), are available from the American Type Culture Collection (ATCC; Bethesda, Md.) and may be chosen to ensure the correct modification and processing of the foreign protein.

For long-term, high-yield production of recombinant proteins, stable expression is preferred. For example, cell lines which stably express the multimeric Fv-antibody may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include, but are not limited to, the herpes simplex virus thymidine kinase (Wigler, M. et al. (1977) Cell 11:223-32) and adenine phosphoribosyltransferase (Lowy, I. et al. (1980) Cell 22:817-23) genes which can be employed in tk.sup.- or aprt.sup.- cells, respectively. Also, antimetabolite, antibiotic or herbicide resistance can be used as the basis for selection; for example, dhfr which confers resistance to methotrexate (Wigler, M. et al. (1980) Proc. Natl. Acad. Sci. 77:3567-70); npt, which confers resistance to the aminoglycosides neomycin and G-418 (Colbere-Garapin, F. et al (1981) J. Mol. Biol. 150:1-14) and als or pat, which confer resistance to chlorsulfuron and phosphinotricin acetyltransferase, respectively (Murry, supra). Additional selectable genes have been described, for example, trpB, which allows cells to utilize indole in place of tryptophan, or hisD, which allows cells to utilize histinol in place of histidine (Hartman, S. C. and R. C. Mulligan (1988) Proc. Natl. Acad. Sci. 85:8047-51). Recently, the use of visible markers has gained popularity with such markers as anthocyanins, .beta. glucuronidase and its substrate GUS, and luciferase and its substrate luciferin, being widely used not only to identify transformants, but also to quantify the amount of transient or stable protein expression attributable to a specific vector system (Rhodes, C. A. et al. (1995) Methods Mol. Biol. 55:121-131).

A particular expression vektor is pSKK2-scFv_{L18}anti-CD3-LL-scFv_{L10}anti-CD19(pSKK2-scFv3LL Db19) (deposited with the DSMZ according to the Budapest Treaty under DSM 14470 at August, 22 2001 or pSKK2-scFv_{L18}anti-CD19-LL-scFv_{L10}anti-CD3(pSKK2-scFv19LL Db3) (deposited with the DSMZ according to the Budapest Treaty under DSM 14471 at August 22, 2001.

The present invention also relates to a pharmaceutical composition containing a multimeric Fv-antibody of the present invention, a DNA sequence or an expression vector, preferably combined with suitable pharmaceutical carriers. Examples of suitable pharmaceutical carriers are well known in the art and include phosphate buffered saline solutions, water, emulsions, such as oil/water emuslions, various types of wetting agents, sterile solutions etc.. Such carriers can be formulated by conventional methods and can be administered to the subject at a suitabel dose. Administration of the suitable compositions may be effected by different ways, e.g. by intravenous, intraperetoneal, subcutaneous, intramuscular, topical or intradermal administration. The route of administration, of course, depends on the nature of the disease, e.g. tumor, and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of the disorder, general health and other drugs being administered concurrently.

Preferred medical uses of the compounds of the present invention are: (a) the treatment of a viral, bacterial, tumoral or prion related diseases, (b) the agglutination of red blood cells, (c) linking cytotoxic cells, e.g. T or Natural killer cells of the immune system to tumor cells, or (d) linking activating cytokines, preferably IL-1, IL-2, IFNγ, TNFα or GM-CSF, cytotoxic substances (e.g doxorubicin, cyclosporin, α-Amanitin or a protease, preferably Granzyme B, to a target cell.

A further object of the present invention is the use of a multimeric Fv-antibody of the present invention for diagnosis. For use in the diagnostic research, kits are also provided by the present invention said kits comprising a multimeric antibody of the present invention. The Fv-antibody can be detectably labeled. In a preferred embodiment, said kit allows diagnosis by ELISA and contains the Fv-antibody bound to a solid support, for example, a polystyrene microtiter dish or nitrocellulose paper, using techniques known in the art. Alternatively, said kit is based on a RIA and contains said Fv-antibody marked with a radioactive isotope. In a preferred embodiment of the kit of the invention the antibody is labelled with enzymes, fluorescent compounds, luminescent compounds, ferromagnetic probes or radioactive compounds.

The present invention is further described with regard to the figures.

### Brief description of the drawings:

### FIGURES 1, 2 and 3: Schemes of the multimeric Fv molecules depending on the particular antibody domains and the length of the peptide linker LL between the variable domains that comprise the multimerization motif

*Abbreviations* L0: The V_{H} and V_{L} domains are directly connected without intervening linker peptide; L1: linker sequence coding for Ser residue; L10: linker sequence coding for SerAlaLysThrThrProLysLeuGlyGly polypeptide connecting V_{H} and V_{L} domains; LL: linker sequence coding for (Gly₄Ser)₄ polypeptide connecting hybrid scFv fragments; L18: linker sequence coding for SerAlaLysThrThrProLysLeuGluGluGlyGluPheSerGluAlaArgVal polypeptide connecting V_{H} and V_{L} domains.

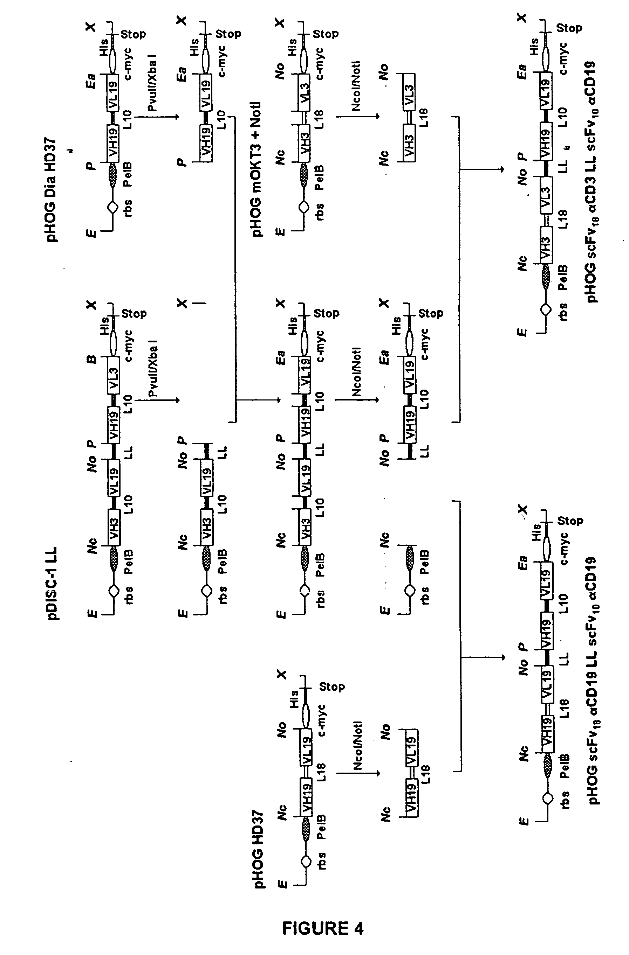

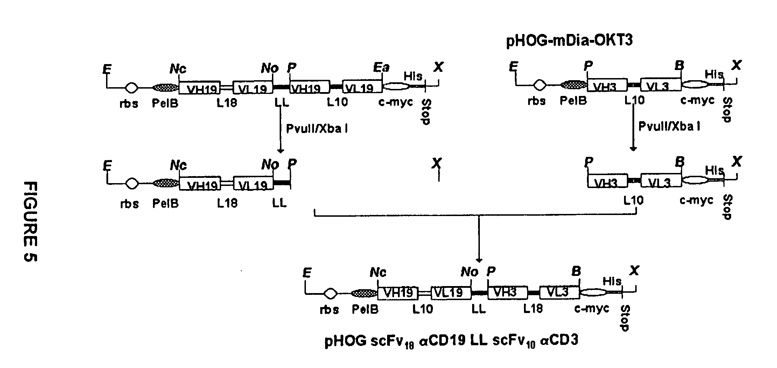

### FIGURES 4 and 5: Scheme of constructing the plasmids pHOG scFv₁₈αCD3-LL-scFv₁₀αCD19 and pHOG scFv₁₈αCD19-LL-scFv₁₀αCD3

*Abbreviations* c-myc: sequence coding for an epitope recognized by mAb 9E10; His: sequence coding for six C-terminal histidine residues; PelB: signal peptide sequence of the bacterial pectate lyase (PelB leader); rbs: ribosome binding site; Stop: stop codon (TAA); V_{H} and V_{L}: variable region of the heavy and light chain; L10: linker sequence coding for SerAlaLysThrThrProLysLeuGlyGly polypeptide connecting V_{H} and V_{L} domains; LL: linker sequence coding for (Gly₄Ser)₄ polypeptide connecting hybrid scFv-fragments; L18: linker sequence coding for SerAlaLysThrThrProLysLeuGluGluGlyGluPheSerGluAlaArgVal polypeptide connecting V_{H} and V_{L} domains; B: BamHI, Ea: EagI, E: EcoRI, Nc: NcoI; N. NotI, P: PvuII, X: XbaI.

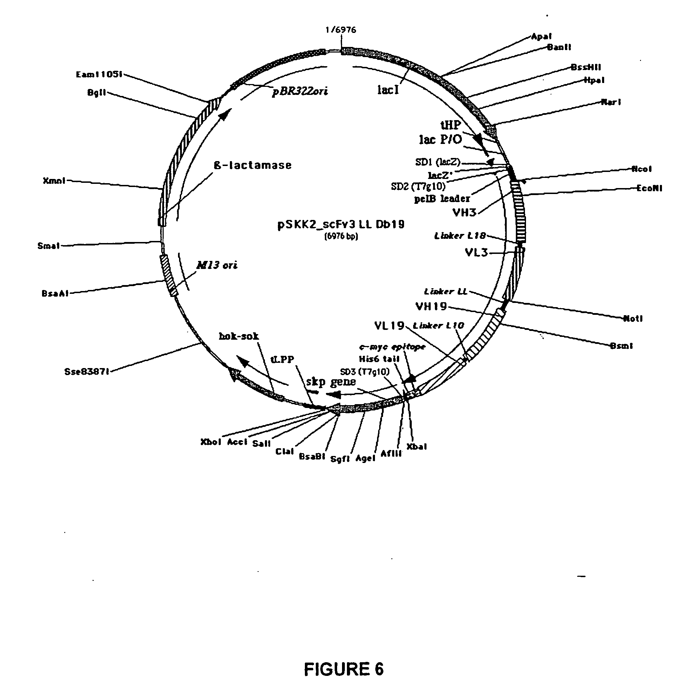

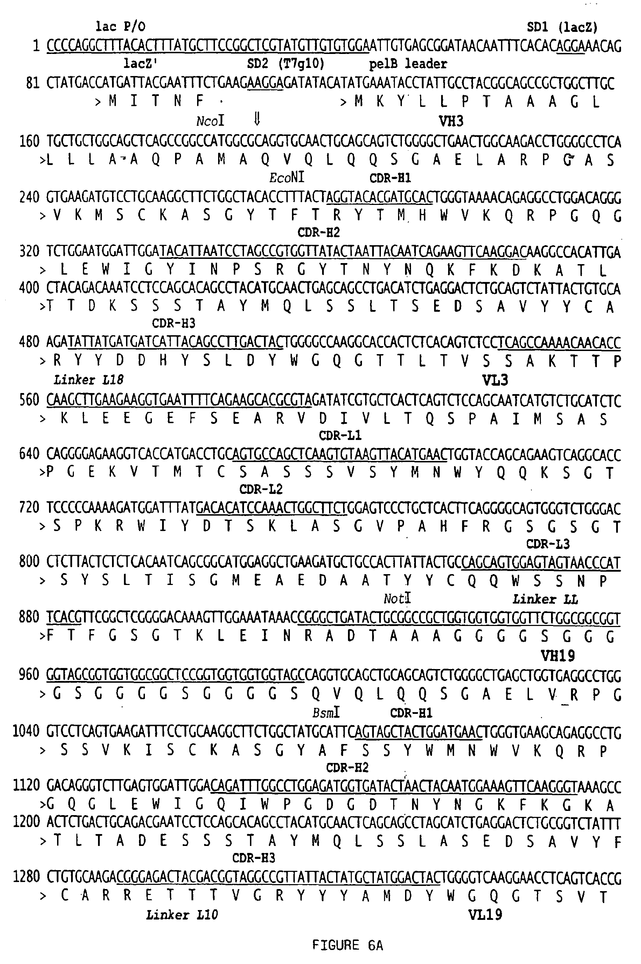

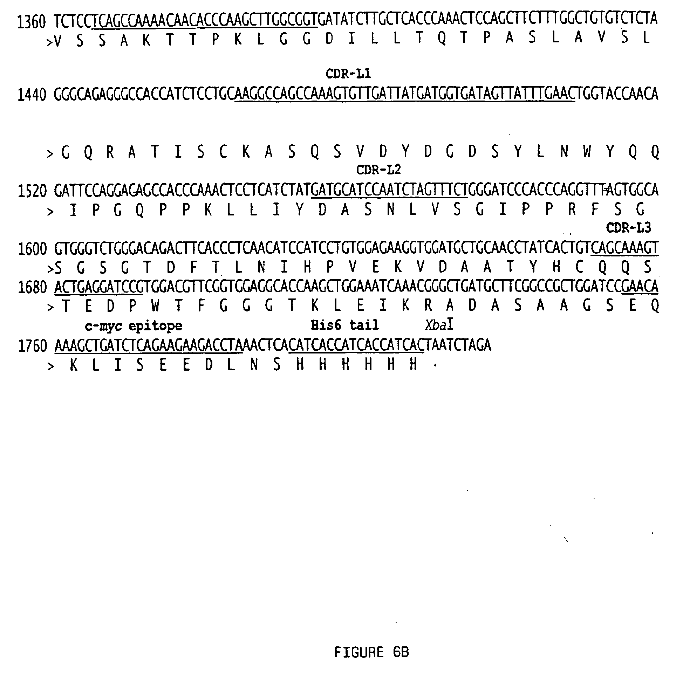

### FIGURE 6: Nucleotide and deduced amino acid sequences of the plasmid pSKK2 scFv3-LL-Db19

*Abbreviations* His6 tail: sequence coding for six C-terminal histidine residues; β-lactamase: gene encoding β-lactamase that confers resistance to ampicillin resistance; bp: base pairs; c-myc epitope: sequence coding for an epitope recognized by mAb 9E10; Lac P/0: wt *lac* operon promoter/operator; Pel B leader: signal peptide sequence of the bacterial pectate lyase; V_{H} and H_{L}: variable region of the heavy and light chain; L10: linker sequence coding for SerAlaLysThrThrProLysLeuGlyGly polypeptide connecting V_{H} and V_{L} domains; LL: linker sequence coding for (Gly₄Ser)₄ polypeptide connecting hybrid scFv-fragments; LI8: linker sequence coding for SerAlaLysThrThrProLysLeuGluGluGlyGluPheSerGluAlaArgVal polypeptide connecting V_{H} and V_{L} domains; rbs: ribosome binding site; V_{H} and V_{L}: variable region of the heavy and light chain; hok-sok: plasmid stabilizing DNA locus; lacI: gene coding for lac-repressor; lac P/0: wt *lac* operon promoter/operator; lacZ': gene coding for α-peptide of β-galactosidase; skp gene: gene encoding bacterial periplasmic factor Skp/OmpH; tLPP: nucleotide sequence of the lipoprotein terminator; *M13* ori: origin of the DNA replication; *pBR322*ori: origin of the DNA replication; tHP: strong terminator of transcription; SD1: ribosome binding site (Shine Dalgarno) derived from *E. coli* lacZ gene (lacZ); SD2 and SD3: Shine Dalgarno sequence for the strongly expressed T7 gene 10 protein (T7g10).

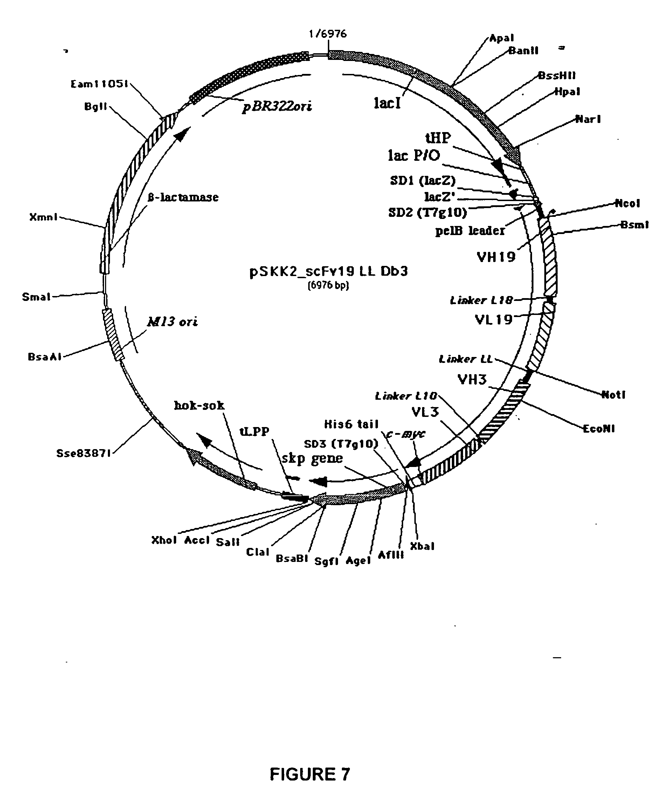

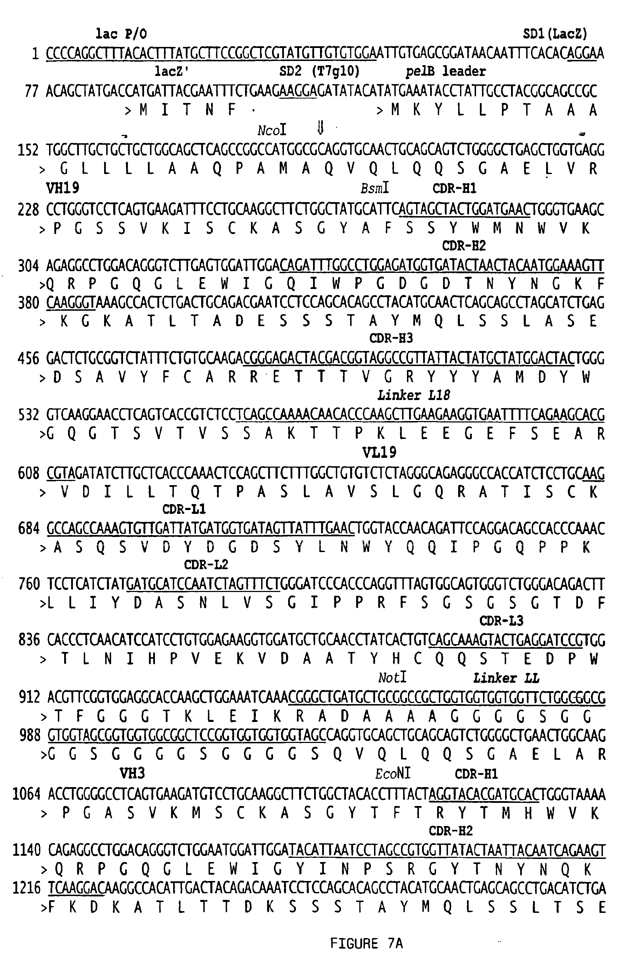

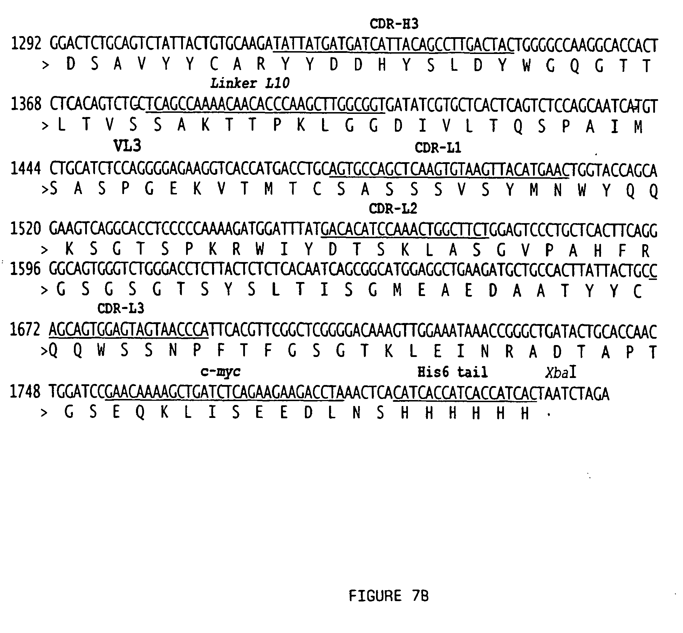

### FIGURE 7: Nucleotide and deduced amino acid sequences of the plasmid pSKK2 scFv19-LL-Db3

*Abbreviations* His6 tail: sequence coding for six C-terminal histidine residues; β-lactamase: gene encoding β-lactamase that confers resistance to ampicillin resistance; bp: base pairs; c-myc epitope: sequence coding for an epitope recognized by mAb 9E10; Lac P/0: wt *lac* operon promoter/operator; PelB leader: signal peptide sequence of the bacterial pectate lyase; V_{H} and V_{L}: variable region of the heavy and light chain; L10: linker sequence coding for SerAlaLysThrThrProLysLeuGlyGly polypeptide connecting V_{H} and V_{L} domains; LL: linker sequence coding for (Gly4Ser)4 polypeptide connecting hybrid scFv-fragments; L18: linker sequence encoding SerAlaLysThrThrProLysLeuGluGluGlyGluPheSerGluAlaArgVal polypeptide connecting V_{H} and V_{L} domains; rbs: ribosome binding site; hok-sok: plasmid stabilizing DNA locus; lacI: gene coding for lac-repressor; lac P/0: wt *lac* operon promoter/operator; lacZ': gene coding for α-peptide of β-galactosidase; skp gene: gene encoding bacterial periplasmic factor Skp/OmpH; tLPP: nucleotide sequence of the lipoprotein terminator; *M13* ori: origin of the DNA replication, *pBR322*ori: origin of the DNA replication; tHP: strong terminator of transcription; SD1: ribosome binding site (Shine Dalgarno) derived from *E. coli* lacZ gene (lacZ); SD2 and SD3: Shine Dalgarno sequence for the strongly expressed T7 gene 10 protein (T7g10).

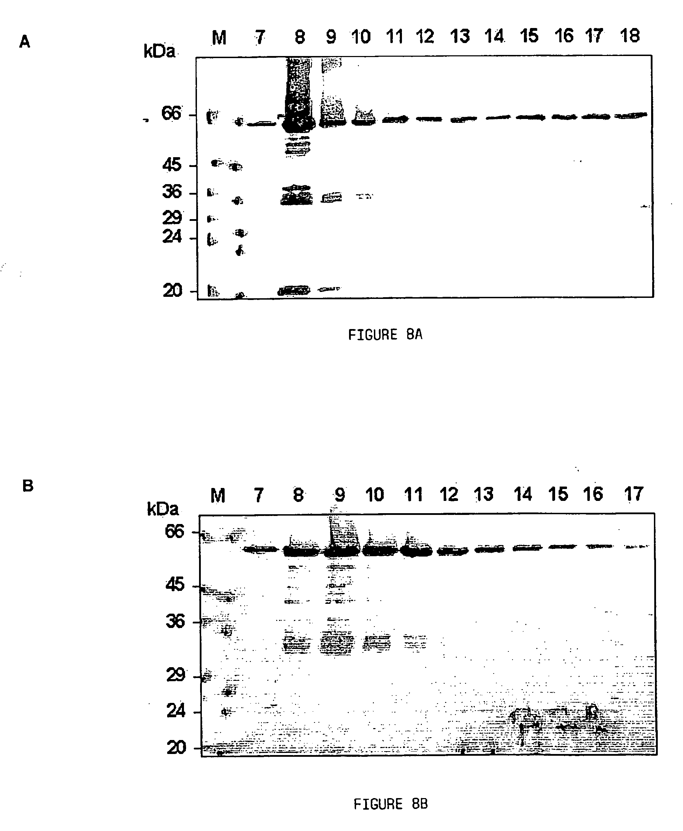

### FIGURE 8: Analysis of protein contents of peaks after IMAC

Electrophoresis was carried out under reducing conditions; Western blot with anti-c-*myc* monoclonal antibody, in the case of scFv3 - Db19 (Figure 8A) and scFv 19 x Db3 (Figure 8B) molecules.

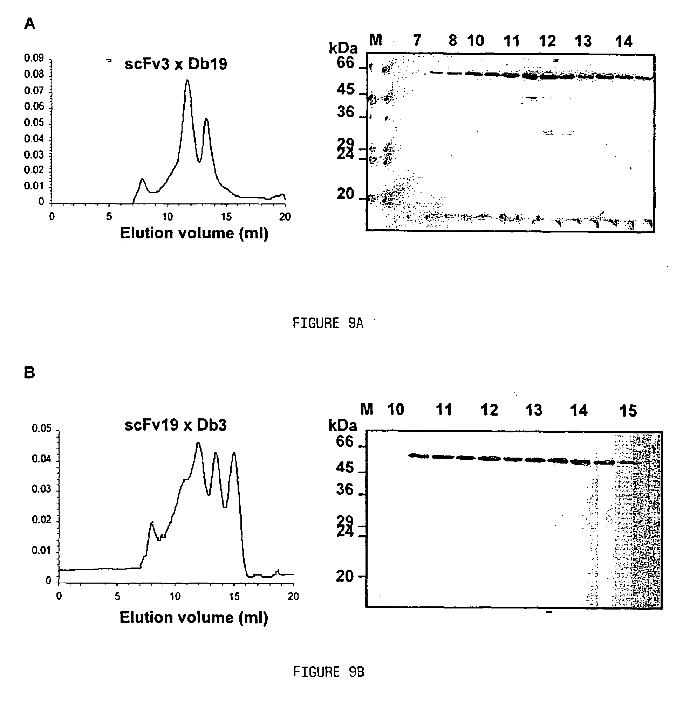

### FIGURE 9: Size-exclusion FPLC chromatography elution profiles

A calibrated Superdex 200 HR10/30 column was used and the analysis of protein contents of peaks by Western blot was carried out with anti-c-*myc* monoclonal antibody, in the case of scFv3 - Db19 (Figure 9A) and scFv19 - Db3 (Figure 9B) molecules.

### FIGURE 10: Size-exclusion FPLC chromatography elution profiles

A calibrated Superdex 200 HR10/3 column for the scFv3 - Db19, scFv19 - Db3, scFv19 - scFv3 and scFv3 - scFv19 molecules was used.

### FIGURE 11: Flow cytometry results on CD3⁺Jurkat and CD19⁺JOK-1 cells

### FIGURE 12: Analysis of cell surface retention on CD19⁺JOK-1 (A,B) and CD3⁺Jurkat cells (C,D) for the scFv3 - scFv19 and scFv3 - Db19 molecules (A,C) and for scFv19 - scFv3 and scFv 19 x Db3 molecules (B,D)

The following Examples illustrate the invention.

### Example 1: Construction of the plasmids pSKK2 scFvL₁₈anti-CD3-LL-scFvL₁₀anti-CD19 (scFv3 - Db19) and pSKK2 scFvL₁₈anti-CD19-LL-scFvL₁₀anti-CD3 (scFv19 - Db3) for expression of multimeric Fv molecules in bacteria

For generation of multimeric Fv constructs, the plasmids pHOG_HD37, pHOG_Dia_HD37, pHOG_mOKT3+NotI and pHOG_Dia_mOKT3 encoding the antibody fragments derived either from hybridoma HD37 specific to human CD19 (Kipriyanov et al., 1996, J. Immunol. Meth. 196, 51-62; Le Gall et al., 1999, FEBS Lett., 453, 164-168) or from hybridoma OKT3 specific to human CD3 (Kipriyanov et al., 1997, Protein Eng. 10, 445-453) were used.

The anti-CD19 scFv_{L10} gene followed by a segment coding for a *c-myc* epitope and a hexahistidinyl tail was cut with PvuII/XbaI from the plasmid pHOG Dia HD37 and recloned into the PvuII/XbaI linearized vector pDISC-1 LL (Kipriyanov et al., 1999, J. Mol. Biol. 293, 41-56)(Figure 4). This hybrid plasmid was linearized by NcoI/NotI and the gene coding for the scFV_{L10} cut by NcoI/NotI from the plasmid pHOG mOKT3 + NotI was ligated into this plasmid. The plasmid obtained is the pHOG scFv_{L18}αCD3-LL-scFv_{L10}αCD19 (scFv3 - Db19) (Figure 4).

The linearized hybrid plasmid NcoI/NotI was also used for the ligation of the gene coding for the scFv_{L18}αCD19 from the plasmid pHOG HD37, the plasmid obtained is the pHOG scFv_{L18α}CD19-LL-scFv_{L10}αCD19 (scFv19 x Db19) (Figure 4). This plasmid was linearized by PvuII/XbaI and the scFv_{L10} gene followed by a segment coding for a *c-myc* epitope and a hexahistidinyl tail was cut with PvuII/XbaI from the plasmid pHOG mDia OKT3. The plasmid obtained is the pHOG scFv_{L18}αCD19-LL-scFv_{L10}αCD3 (scFv19 - Db3) (Figure 5).

To increase the yield of functional antibody fragments in the bacterial periplasm, an optimized expression vector pSKK2 was generated. This vector was constructed on the base of-plasmid pHKK (Horn, 1996, Appl. Microbiol. Biotechnol., 46, 524-532) containing *hok*/*sok* plasmid-free cell suicide system (Thisted et al., 1994, EMBO J., 13, 1950-1956). First, the gene coding for hybrid scFv V_{H}3-V_{L}19 was amplified by PCR from the plasmid pHOG3-19 (Kipriyanov et al., 1998, Int. J. Cancer 77, 763-772) using the primers 5-NDE, 5'-GATATACATATGAAATACCTATTGCCTACGGC, and 3-AFL, 5'-CGAATTCTTAAGTTAGCACAGGCCTCTAGAGACACACAGATCTTTAG. The resulting 921 bp PCR fragment was digested with *Nde*I and *Afl*II and cloned into the *Nde*I/*Afl*II linearized plasmid pHKK, generating the vector pHKK3-19. To delete an extra *Xba*I site, a fragment of pHKK plasmid containing 3'-terminal part of the *lac*I gene (encodes the *lac* repressor), the strong transcriptional terminator t_{HP} and wild-type *lac* promoter/operator was amplified by PCR using primers 5-NAR, 5'-CACCCTGGCGCCCAATACGCAAACCGCC, and 3-NDE, 5'-GGTATTTCATATGTATATCTCCTTCTTCAGAAATTCGTAATCATGG. The resulting 329 bp DNA fragment was digested with *Nar*I and *Nde*I and cloned into *Nar*I/*Nde*I linearized plasmid pHKK3-19 generating the vector pHKK□Xba. To introduce a gene encoding the Skp/OmpH periplasmic factor for higher recombinant antibody production (Bothmann and Plückthun, 1998, Nat. Biotechnol., 16, 376-380), the *skp* gene was amplified by PCR with primers skp-3, 5'-CGAATTCTTAAGAAGGAGATATACATATGAAAAAGTGGTTATTAGCTGCAGG and skp-4, 5'-CGAATTCTCGAGCATTATTTAACCTGTTTCAGTACGTCGG using as a template the plasmid pGAH317 (Holck and Kleppe, 1988, Gene, 67, 117-124). The resulting 528 bp PCR fragment was digested with *Afl*II and *Xho*I and cloned into the *Afl*II/*Xho*I digested plasmid pHKK□Xba resulting in the expression plasmid pSKK2.

The plasmids pHOG scFv_{L18}αCD3-LL-scFv_{L10}αCD19 (scFv3 - Dbl9) and pHOG scFv_{L18}αCD19-LL-scFv_{L10}αCD3 (scFv19 - Db3) were cut by NcoI/XbaI and ligated in the NcoI/XbaI linearized plasmid pSKK2. The resulting plasmids are pSKK2 scFv_{L18}αCD3-LL-scFv_{L10}αCD19 and pSKK2 scFv_{L18}αCD19-LL-scFv_{L10}αCD3. The complete nucleotide and amino acid sequences are given in Figures 6 and 7, respectively.

### Example 2: Expression and purification of the multimeric Fv molecules in bacteria

The *E. coli* K12 strain RV308 (Maurer et al., 1980, J. Mol. Biol. 139, 147-161) transformed with the expression plasmids pSKK2 scFV_{Lle}ocCD3-LL-scFV_{L10}αCD19 and pSKK2 scFv_{L18}αCD19-LL-scFv_{L10}αCD3 were grown overnight in 2xYT medium with 50 µg/ml ampicillin and 100 mM glucose (2xYT_{GA}) at 28°C. Dilutions (1:50) of the overnight cultures in 2xYT_{GA} were grown as flask cultures at 28°C with shaking at 200 rpm. When cultures reached OD₆₀₀ = 0.8, bacteria were pelleted by centrifugation at 5,000 x g for 10 min and 20°C and resuspended in the same volume of fresh YTBS medium (2xYT containing 1 M sorbitol and 2.5 mM glycine betaine; Blacwell & Horgan, 1991, FEBS Letters. 295, 10-12) containing 50 µg/ml ampicillin. IPTG was added to a final concentration of 0.2 mM and growth was continued at 20°C for 18-20 h. Cells were harvested by centrifugation at 9,000 x g for 20 min and 4°C. To isolate soluble periplasmic proteins, the pelleted bacteria were resuspended in 5% of the initial volume of ice-cold 50 mM Tris-HCl, 20% sucrose, 1 mM EDTA, pH 8.0. After a 1 h incubation on ice with occasional stirring, the spheroplasts were centrifuged at 30,000 x g for 30 min and 4°C leaving the soluble periplasmic extract as the supernatant and spheroplasts plus the insoluble periplasmic material as the pellet. The periplasmic fractions were dialyzed against start buffer (50 mM Tris-HCl, 1 M NaCl, 50 mM Imidazole pH 7.0) at 4°C. The dialyzed solution containing recombinant product was centrifuged at 30,000 x g for 30 min and 4°C. The recombinant product- was concentrated by ammonium sulfate precipitation (final concentration 70% of saturation). The protein precipitate was collected by centrifugation (10,000 x g, 4°C, 40 min) and dissolved in 10% of the initial volume of 50 mM Tris-HCl, 1 M NaCl, pH 7.0. Immobilized metal affinity chromatography (IMAC) was performed at 4°C using a 5 ml column of Chelating Sepharose (Pharmacia) charged with Cu²⁺ and equilibrated with 50 mM Tris-HCl, 1 M NaCl, pH 7.0 (start buffer). The sample was loaded by passing the sample over the column. It was then washed with twenty column volumes of start buffer followed by start buffer containing 50 mM imidazole until the absorbency (280 nm) of the effluent was minimal (about thirty column volumes). Absorbed material was eluted with 50 mM Tris-HCl, 1 M NaCl, 250 mM imidazole, pH 7.0. The elution fractions containing the multimeric Fv-molecules were identified by Western-blot analysis using anti-c-*myc* Mab 9E10 performed as previously described (Kipriyanov et al., 1994, Mol. Immunol. 31, 1047-1058) as illustrated in Figure 8A for scFv3 - Db19 and Figure 8B for scFv19 - Db3.

The positive fractions were collected and concentrated on Ultrafree-15 centrifugal filter device (Millipore Corporation, Eschborn, Germany) until 0,5 ml.

Further purification of the multimeric Fv-molecules was done by size-exclusion FPLC on a Superdex 200 HR10/30 column (Pharmacia) in PBSI (15 mM sodium phosphate, 0,15 M NaCl, 50 mM Imidazole, pH 7.0). Sample volumes for preparative chromatography were 500 µl and the flow rate was 0.5 ml/min, respectively. The column was calibrated with High and Low Molecular Weight Gel Filtration Calibration Kits (Pharmacia). The elution fractions containing the multimeric Fv-molecules were identified by Western-blot analysis using anti-c-*myc* Mab 9E10 performed as previously described (Kipriyanov et al., 1994, Mol. Immunol. 31, 1047-1058) and the results presented in FIGURE 9A and 9B for scFv3 - Db19 and scFv19 - Db3 molecules, respectively. The fractions were collected and stored individually on ice.

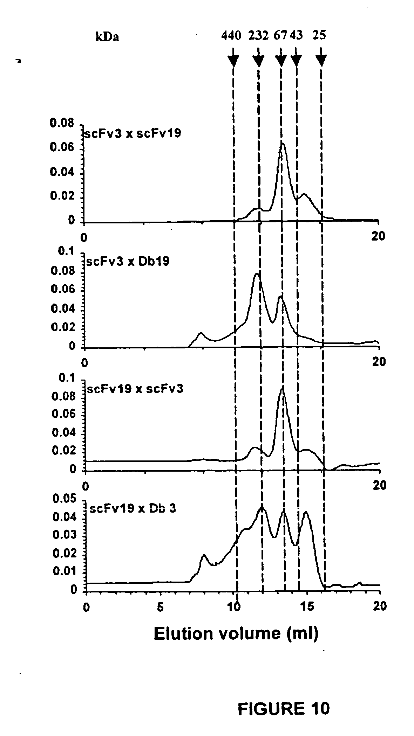

The generated Fv molecules were compared with two scFv-scFv tandems, scFv3 - scFv19 and scFv19 - scFv3 (Figure 10), produced and purified under the same conditions. Figure 10 clearly demonstrates that higher molecular forms were obtained for the scFv3 x Db 19 and scFv 19 x Db3 in comparison with scFv3 x scFv 19 and scFv 19 x scFv3. The main peak for scFv3 - scFv19 and scFv19 - scFv3 molecules correspond to a molecular weight of about 67 kDa, and to about 232 kDa for the scFv3 - Db19 and scFv19 - Db3. The presence of the dimerization motif on the C-terminus of the molecule has a positive effect for the multimerisation of the molecules.

### Example 3: Characterization of the multimeric Fv molecules by flow cytometry

The human CD3⁺/CD19⁻acute T cell leukemia line Jurkat and the CD19⁺/CD3⁻B cell line JOK-1 were used for flow cytometry. In brief, 5 x 10⁵ cells in 50 µl RPMI 1640 medium (GIBCO BRL, Eggenstein, Germany) supplemented with 10% FCS and 0.1% sodium azide (referred to as complete medium) were incubated with 100 µl of a multimeric Fv molecule preparation for 45 min on ice. After washing with complete medium, the cells were incubated with 100 µl of 10 µg/ml anti c-myc MAb 9E10 (IC Chemikalien, Ismaning, Germany) in the same buffer for 45 min on ice. After a second washing cycle, the cells were incubated with 100 µl of FITC-labeled goat anti-mouse IgG (GIBCO BRL) under the same conditions as before. The cells were then washed again and resuspended in 100 µl of 1 µg/ml solution of propidium iodide (Sigma, Deisenhofen, Germany) in complete medium to exclude dead cells. The relative fluorescence of stained cells was measured using a FACScan flow cytometer (Becton Dickinson, Mountain View, CA) or Epics XL flow cytometer systems (Beckman Coulter, Miami, FL).

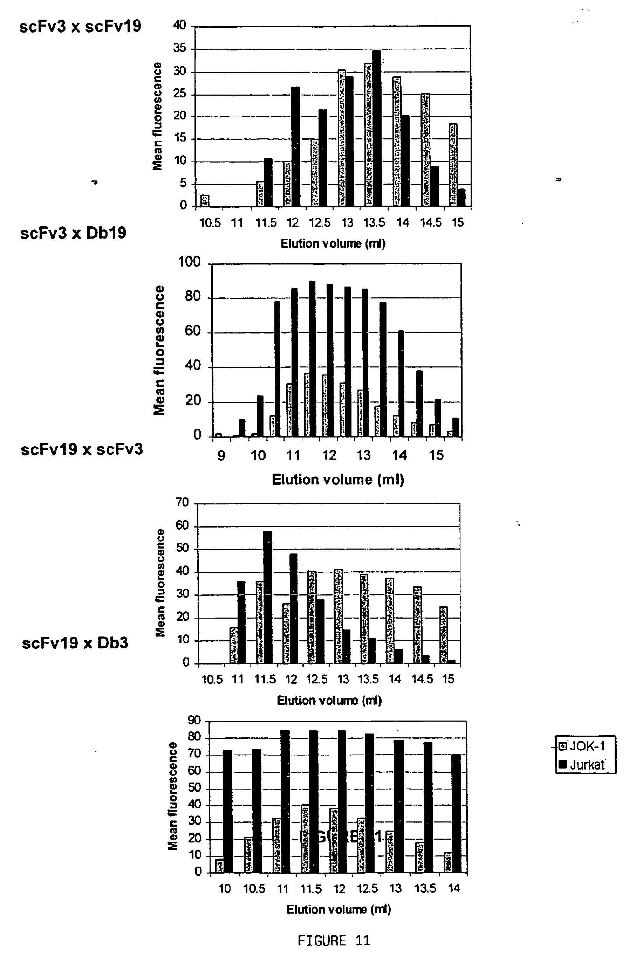

Flow cytometry experiments demonstrated specific interactions with both human CD3⁺Jurkat and the CD19⁺JOK-1 cells for all the multimeric Fv-molecules (Figure 11).

For the CD19 and CD3 binding affinities it was decided to use the fractions corresponding to the monomers for scFv3 x scFv 19 and scFv19 - scFv3, and to the multimers for scFv3 x Db 19 and scFv 19 x Db3.

### Example 4: In vitro cell surface retention assay of the multimeric Fv molecules

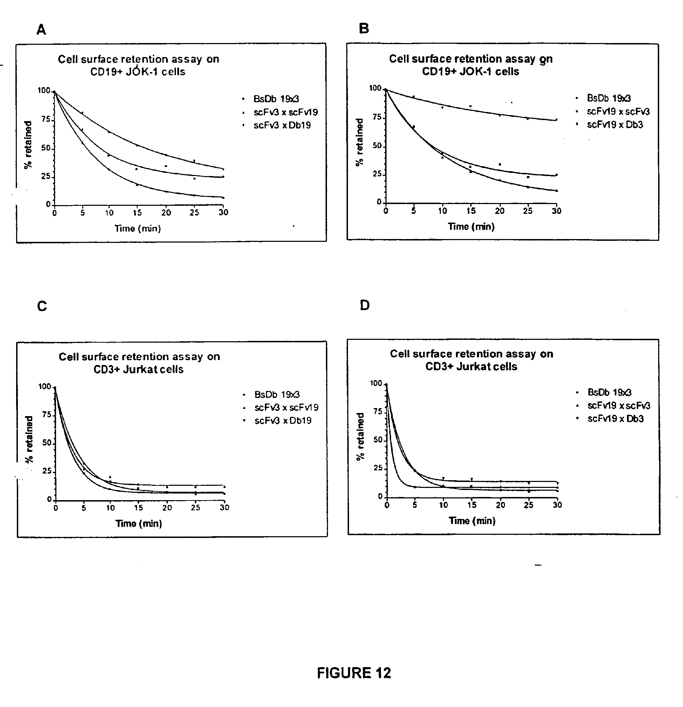

Cell surface retention assays were performed at 37°C essentially as described (Adams et al., 1998, Cancer Res. 58, 485-490) except that the detection of the retained antibody fragments was performed using anti c-*myc* MAb 9E10 followed by FITC-labeled anti-mouse IgG. Kinetic dissociation constant (k_{off}) and the half-life (t_{1/2}) for dissociation of the multimeric Fv-molecules were deduced from a one-phase exponential decay fit of experimental data using "GraphPad" Prism (GraphPad Software, San Diego, CA). For control, the bispecific diabody CD19 x CD3 (BsDb 19x3) described previously (Kipriyanov et al., 1998, Int. J. Cancer 77, 763-777; Cochlovius et al., 2000, J. Immunol. 165u 888-895) was used. The results of the experiments are shown in Figure 12 and summarized on Table 1.

The scFv3 - scFv19 had a relatively short retention half-life (t_{1/2}) on CD19⁺JOK-1 cells, almost two time less than with the t_{1/2} of the BsDb 19x3 (Table 1). In contrast, the scFv3 - Db19 was retained longer on the surface of JOK-1 cells. For the scFv19 - scFv3 the t_{1/2} is in the same range than for the t_{1/2} of the BsDb 19x3. The retention of the scFv3 - Db19 is significantly higher, with t_{1/2} = 65,71 min, in comparison with the others molecules (Table 1). The half-lives of all the multispecific Fv-molecules on the surface of CD3⁺Jurkat cells were relatively short. The length of the linker appeared to have some influence on antigen binding, since the scFv3 - Db19 and scFvl9 - Db3 showed a significantly slower k_{off} from CD19-positive cells than the BsDb 19x3, scFv3 x scFv 19 and scFv19 - scFv3.

**Table 1**

| **Binding kinetics of recombinant bispecific molecules** | | | | | |
|---|---|---|---|---|---|
| Antibody | *k*_{*off*} | *t*_{1/2} | Antibody | *k*_{*off*} | *t*_{1/2} |
| | (s⁻¹/10⁻³) | (min) | | (s⁻¹/10⁻³) | (min) |
| *A. JOK-1 cells (CD3*^{*-*}/*CD19*^{*+*}*)* | | | *B. JOK-1 cells (CD3*^{*-*}/*CD19*^{*+*}*)* | | |
| BsDb 19x3 | 0.9945 | 11.62 | BsDb 19x3 | 0.1512 | |
| 10.68 | | | | | |
| scFv3-scFv19 | 1.814 | 6.368 | scFv19-scFv3 | 0.05547 | 8.622 |
| scFv3-Db19 | 0.6563 | 17.6 | scFv19-Db3 | 0.01171 | |
| 65.71 | | | | | |

| *C. Jurkat cells (CD3*^{*+*}/*CD19*^{*-*}*)* | | | *D. Jurkat cells (CD3*^{*+*}/*CD19*^{*-*}*)* | | |
|---|---|---|---|---|---|
| BsDb 19x3 | 4.268 | 2.707 | BsDb 19x3 | 4.268 | 2.707 |
| scFv3-scFv19 | 2.912 | 3.967 | scFv19-scFv3 | 6.91 | 1.672 |
| scFv3-Db19 | 3.161 | 3.655 | scFv19-Db3 | 3.4 | 3.394 |

## Claims

1. A multimeric Fv-antibody, wherein each monomer of the Fv-antibody comprises the following features:
(a) two neighboring variable domains that form an antigen-binding V_{H}-V_{L} or V_{L}-V_{H} scFv unit; these two, first and second, variable domains are linked by a peptide linker of at least 12 amino acid residues;and
(b) two other neighboring variable domains that are non-covalently bound to two variable domains of another monomer of the Fv-antibody resulting in the formation of two additional antigen binding sites to form the multimerization motif; these two, third and fourth, variable domains of each monomer are linked by a peptide linker consisting of a maximun of 10 amino acid residue.

2. The multimeric Fv-antibody of claim 1, wherein a further feature is that the antigen-binding V_{H}-V_{L} or V_{L}-V_{H} scFv unit formed by the two neighboring domains of one monomer is linked to the other variable domains of the multimerization motif by a peptide linker of at least 15 amino acid residues.

3. The multimeric Fv-antibody of claim 1 or 2, wherein said monomers comprise four variable domains and wherein the third and fourth variable domain of the monomers are linked by a peptide linker of 5 or less amino acid residues.

4. The multimeric Fv-antibody of claim 3, wherein the third and fourth domain of the monomers are linked directly without intervening amino acid residues.

5. The multimeric Fv-antibody of any one of claims 1 to 4, wherein the second and third variable domain of the monomers are linked by a peptide linker consisting of at least 20 amino acid residues.

6. The multimeric Fv-antibody of any one of claims 1 to 5, wherein the third and/or fourth variable domain of the monomers are shortened by at least one amino acid residue at their N- and/or C-terminus.

7. The multimeric Fv-antibody of any one of claims 1 to 6, wherein the order of domains of a monomer is V_{H}-V_{L}.

8. The multimeric Fv-antibody of any one of claims 1 to 6, wherein the order of domains of a monomer is V_{L}-V_{H}.

9. The multimeric Fv-antibody of any one of claims 1 to 8, wherein the non-covalent binding of at least one pair of variable domains is strengthened by at least one disulfide bridge.

10. The multimeric Fv-antibody of any one of claims 1 to 9, which is a tetravalent dimer, hexavalent trimer or octavalent tetramer.

11. The multimeric Fv-antibody of any one of claims 1 to 10, which is a bispecific or trispecific or tetraspecific antibody.

12. The multimeric Fv-antibody of any one of claims 1 to 11, wherein at least one monomer is linked to a biologically active substance, a chemical agent, a petide, a protein or a drug.

13. The multimeric Fv-antibody of any one of claims 1 to 10 or 12, which is a monospecific antibody capable of specifically binding the CD19 antigen of B-lymphocytes or the CEA antigen.

14. The multimeric Fv-antibody of any one of claims 1 to 12, which is a bispecific antibody capable of specifically binding:
(a) CD19 and the CD3 complex of the T-cell receptor;
(b) CD19 and the CD5 complex of the T-cell receptor;
(c) CD19 and the CD28 antigen on T-lymphocytes;
(d) CD19 and CD16 on natural killer cells, macrophages and activated monocytes;
(e) CEA and CD3;
(f) CEA and CD28; or
(g) CEA and CD16.

15. A process for the preparation of a multimeric Fv-antibody of any one of claims 1 to 14, wherein (a) DNA sequences encoding the peptid linkers are ligated with the DNA sequences encoding the variable domains such that the peptide linkers connect the variable domains resulting in the formation of a DNA sequence encoding a monomer of the multivalent multimeric Fv-antibody and (b) the DNA sequences encoding the various monomers are expressed in a suitable expression system.

16. A DNA sequence encoding a multimeric Fv-antibody of any one of claims 1 to 14.

17. An-expression vector containing the DNA sequence of claim 16.

18. The expression vector of claim 17, which is pSKK2-scFv_{L18}anti-CD3-LL-scFv_{L10}anti-CD19 also named pSKK2-scFv3LL Db19 and deposited as DSM 14470 or pSKK2-scFv_{L18}anti-CD19-LL-scFv_{L10}anti-CD3 also named pSKK2-scFv19LL Db3 and deposited as DSM 14471.

19. A host cell containing the expression vector of claim 16 or 18.

20. A pharmaceutical composition containing a multimeric Fv-antibody of any one of claims 1 to 14.

21. A multimeric Fv-antibody of any one of claims 1 to 14 for the use as a diagnostic reagens.

22. Use of a multimeric Fv-antibody of any one of claims 1 to 14, for the preparation of a pharmaceutical composition for (a) the treatment of a viral, bacterial, tumoral or prion related disease, (b) the agglutination of red blood cells, (c) linking cytotoxic cells of the immune system to tumor cells, or (d) linking activating cytokines, cytotoxic substances or a protease to a target cell.

23. A diagnostic kit containing a multimeric Fv-antibody of any one of claims 1 to 14.

## Patentansprüche

1. Multimerer Fv-Antikörper, wobei jedes Monomer des Fv-Antikörpers die folgenden Merkmale umfasst:
(a) zwei benachbarte variable Domänen, die eine Antigen bindende V_{H-}V_{L}- oder V_{L}-V_{H}-scFv-Einheit bilden; diese beiden, erste und zweite, variablen Domänen werden durch einen Peptid-Linker mit mindestens 12 Aminosäureresten verbunden; und
(b) zwei andere benachbarte variable Domänen, die nicht kovalent an zwei variable Domänen eines anderen Monomers des Fv-Antikörpers gebunden sind, was zur Bildung von zwei zusätzlichen Antigenbindungsstellen führt, um das Multimerisierungsmotiv zu ergeben; diese beiden, dritte und vierte, variablen Domänen jedes Monomers sind durch einen Peptid-Linker verbunden, der aus maximal 10 Aminosäureresten besteht.

2. Multimerer Fv-Antikörper nach Anspruch 1, wobei ein weiteres Merkmal darin besteht, dass die Antigen bindende V_{H}-V_{L}- oder V_{L}-V_{H}-scFv-Einheit, die durch die beiden benachbarten Domänen des einen Monomers gebildet werden, mit den anderen variablen Domänen des Multimerisierungsmotivs über einen Peptid-Linker von mindestens 15 Aminosäureresten verbunden ist.

3. Multimerer Fv-Antikörper nach Anspruch 1 oder 2, wobei die Monomeren vier variable Domänen umfassen und wobei die dritte und vierte variable Domäne der Monomeren mittels eines Peptid-Linkers von 5 oder weniger Aminosäureresten verbunden ist.

4. Multimerer Fv-Antikörper nach Anspruch 3, wobei die dritte und vierte Domäne der Monomere ohne intervenierende Aminosäurereste direkt verbunden sind.

5. Multimerer Fv-Antikörper nach einem der Ansprüche 1 bis 4, wobei die zweite und dritte variable Domäne der Monomeren mittels eines Peptid-Linkers verbunden sind, der aus mindestens 20 Aminosäureresten besteht.

6. Multimerer Fv-Antikörper nach einem der Ansprüche 1 bis 5, wobei die dritte und/oder vierte variable Domäne der Monomeren um mindestens einen Aminosäurerest an ihrem N- und/oder C-Terminus gekürzt ist.

7. Multimerer Fv-Antikörper nach einem der Ansprüche 1 bis 6, wobei die Reihenfolge von Domänen eines Monomers V_{H}-V_{L} ist.

8. Multimerer Fv-Antikörper nach einem der Ansprüche 1 bis 6, wobei die Reihenfolge von Domänen eines Monomers V_{L}-V_{H} ist.

9. Multimerer Fv-Antikörper nach einem der Ansprüche 1 bis 8, wobei die nicht kovalente Bindung von mindestens einem Paar von variablen Domänen durch mindestens eine Disulfidbrücke verstärkt ist.

10. Multimerer Fv-Antikörper nach einem der Ansprüche 1 bis 9, welcher ein tetravalentes Dimer, hexavalentes Trimer oder oktavalentes Tetramer ist.

11. Multimerer Fv-Antikörper nach einem der Ansprüche 1 bis 10, welcher ein bispezifischer oder trispezifischer oder tetraspezifischer Antikörper ist.

12. Multimerer Fv-Antikörper nach einem der Ansprüche 1 bis 11, wobei mindestens ein Monomer mit einer biologisch aktiven Substanz, einem chemischen Mittel, einem Peptid, einem Protein oder einem Medikament verbunden ist.

13. Multimerer Fv-Antikörper nach einem der Ansprüche 1 bis 10 oder 12, welcher ein monospezifischer Antikörper ist, der in der Lage ist, das CD19-Antigen von B-Lymphozyten oder das CEA-Antigen spezifisch zu binden.

14. Multimerer Fv-Antikörper nach einem der Ansprüche 1 bis 12, welcher ein bispezifischer Antikörper ist, der in der Lage ist:
(a) CD19 und den CD3-Komplex des T-Zell-Rezeptors;
(b) CD19 und den CD5-Komplex des T-Zell-Rezeptors;
(c) CD19 und das CD28-Antigen auf T-Lymphozyten;
(d) CD19 und CD16 auf natürlichen Killerzellen, Makrophagen und aktivierten Monozyten;
(e) CEA und CD3;
(f) CEA und CD28; oder
(g) CEA und CD16
spezifisch zu binden.

15. Verfahren zur Herstellung eines multimeren Fv-Antikörpers nach einem der Ansprüche 1 bis 14, wobei (a) DNA-Sequenzen, die die Peptidlinker kodieren, mit den DNA-Sequenzen, die die variablen Domänen kodieren, so ligiert werden, dass die Peptid-Linker die variablen Domänen verbinden, was zur Bildung einer DNA-Sequenz führt, die ein Monomer des multivalenten multimeren Fv-Antikörpers kodiert, und (b) die DNA-Sequenzen, die die verschiedenen Monomeren kodieren, in einem geeigneten Expressionssystem exprimiert werden.

16. DNA-Sequenz, die einen multimeren Fv-Antikörper nach einem der Ansprüche 1 bis 14 kodiert.

17. Expressionsvektor, enthaltend die DNA-Sequenz nach Anspruch 16.

18. Expressionsvektor nach Anspruch 17, welcher pSKK2-scFv_{L18}anti-CD3-LL-scFv_{L10}anti-CD19, auch bezeichnet als pSKK2-scFv3LL Db19 und hinterlegt als DSM 14470, oder pSKK2-scFv_{L18}anti-CD19-LL-scFv_{L10}anti-CD3, auch bezeichnet als pSKK2-scFv19LL Db3 und hinterlegt als DSM 14471, ist.

19. Wirtszelle, enthaltend den Expressionsvektor nach Anspruch 16 oder 18.

20. Pharmazeutische Zusammensetzung, enthaltend einen multimeren Fv-Antikörper nach einem der Ansprüche 1 bis 14.

21. Multimerer Fv-Antikörper nach einem der Ansprüche 1 bis 14 zur Verwendung als diagnostisches Reagenz.

22. Multimerer Fv-Antikörper nach einem der Ansprüche 1 bis 14, zur Herstellung einer pharmazeutischen Zusammensetzung zur (a) Behandlung einer viralen, bakteriellen, tumoralen oder prionbezogenen Erkrankung, (b) Agglutination von roten Blutzellen, (c) Verbindung von zytotoxischen Zellen des Immunsystems mit Tumorzellen, oder (d) Binden von aktivierenden Zytokinen, zytotoxischen Substanzen oder einer Protease an eine Zielzelle.

23. Diagnostischer Kit, enthaltend einen multimeren Fv-Antikörper nach einem der Ansprüche 1 bis 14.

## Revendications

1. Anticorps multimère Fv dans lequel chaque monomère de l'anticorps Fv comprend les caractéristiques suivantes :
(a) deux domaines variables voisins qui forment une unité scFv V_{H}-V_{L} ou V_{L}-V_{H} liant antigène, ces deux, premier et second, domaines variables étant liés par un liant peptide comprenant des restes de 12 acides aminés ; et
(b) deux autres domaines variables voisins liés de manière non-covalente à deux domaines variables d'un autre monomère de l'anticorps Fv résultant de la formation de deux sites additionnels de liaison antigène pour former le motif de multimérisation, ces deux, troisième et quatrième, domaines variables de chaque monomère étant liés à l'aide d'un liant peptide consistant en des restes d'au maximum 10 acides aminés.

2. Anticorps multimère Fv selon la revendication 1, **caractérisé en ce qu'**une autre caractéristique est que l'unité scFv V_{H}-V_{L} ou V_{L}-V_{H} liant antigène formée par les deux domaines voisins d'un monomère est liée aux autres domaines variables du motif de multimérisation par un liant peptide de restes d'au moins 15 acides aminés.

3. Anticorps multimère Fv selon la revendication 1 ou 2, **caractérisé en ce que** lesdits monomères comportent quatre domaines variables et **en ce que** les troisième et quatrième domaines des monomères sont liés par un liant peptide de restes de 5 ou moins acides aminés.

4. Anticorps multimère Fv selon la revendication 3, **caractérisé en ce que** les troisième et quatrième domaines des monomères sont liés directement sans intervention de restes d'acides aminés.

5. Anticorps multimère Fv selon l'une des revendications 1 à 4, **caractérisé en ce que** les second et troisième domaines variables des monomères sont liés par un liant peptide consistant en des restes d'au moins 20 acides aminés.

6. Anticorps multimère Fv selon l'une des revendications 1 à 5, **caractérisé en ce que** les troisième et/ou quatrième domaines variables des monomères sont raccourcis par un reste d'au moins un acide aminé par leur terminaisons N-et/ou C-.

7. Anticorps multimère Fv selon l'une des revendications 1 à 6, **caractérisé en ce que** l'ordre des domaines d'un monomère est V_{H}-V_{L}.

8. Anticorps multimère Fv selon l'une des revendications 1 à 6, **caractérisé en ce que** l'ordre des domaines d'un monomère est V_{L}-V_{H}.

9. Anticorps multimère Fv selon l'une des revendications 1 à 8, **caractérisé en ce que** la liaison non covalente liant au moins une paire de domaines variables est renforcée par au moins un pont disulfide.

10. Anticorps multimère Fv selon l'une des revendications 1 à 9, **caractérisé en ce qu'**il est un dimère tétravalent, un trimère haxavalent ou un tétramère octavalent.

11. Anticorps multimère Fv selon l'une des revendications 1 à 10, **caractérisé en ce qu'**il est un anticorps bi-spécifique, tri-spécifique ou tétra-spécifique.

12. Anticorps multimère Fv selon l'une des revendications 1 à 11, **caractérisé en ce qu'**au moins un monomère est lié à une substance biologiquement active, un agent chimique, un peptide, une protéine ou un médicament.

13. Anticorps multimère Fv selon l'une des revendications 1 à 10 or 12, **caractérisé en ce qu'**il est un anticorps monospécifique apte à lier spécifiquement l'antigène CD19 des lymphocytes B ou l'antigène CEA.

14. Anticorps multimère Fv selon l'une des revendications 1 à 12, **caractérisé en ce qu'**il est un anticorps spécifique apte à lier spécifiquement :
(a) CD19 et le complexe CD3 du récepteur de cellule T,
(b) CD19 et le complexe CD5 du récepteur de cellule T,
(c) CD19 et l'antigène CD28 sur les lymphocytes T,
(d) CD19 et CD16 sur les cellules tueuses naturelles, les macrophages et les monocytes activés,
(e) CEA et CD3,
(f) CEA et CD28, ou
(g) CEA et CD16.

15. Procédé pour la préparation d'un anticorps multimère Fv selon l'une des revendications 1 à 14, **caractérisé en ce que** (a) on lit des séquences ADN encodant les liants peptide avec les séquences ADN encodant les domaines variables de telle façon que les liants peptide relient les domaines variables résultant de la formation d'une séquence ADN encodant un monomère de l'anticorps multimère Fv multivalent et (b) on exprime les séquences ADN encodant les monomères divers dans un système d'expression souhaitable.

16. Séquence ADN encodant un anticorps multimère FV selon l'une des revendications 1 à 14.

17. Vecteur d'expression renfermant la séquence ADN selon la revendication 16.

18. Vecteur d'expression selon la revendication 17 qui est pSKK2-scFv_{L18}anti-CD3-LL-scFv_{L10}anti-CD19, nommé pSFF2-scFv3LL Db19 déposé sous la désignation DSM 14470 ou bien pSKK2-scFv_{L18}anti-CD19-LL-scFv_{L10}anti-CD3, nommé psKK2-scFv19LL Db3 déposé sous la désignation DSM 14471.

19. Cellule hôte contenant le vecteur d'expression selon la revendication 16 ou 18.

20. Composition pharmaceutique contenant un anticorps multimère Fv selon l'une des revendications 1 à 14.

21. Anticorps multimère Fv selon l'une des revendications 1 à 14 appliqué à l'utilisation d'un réactif de diagnostique.

22. Utilisation d'un anticorps multimère Fv selon l'une des revendications 1 à 14 pour la préparation d'une composition pharmaceutique pour (a) le traitement d'une maladie virale, bactérielle, tumorale ou de prion, (b) l'agglutination des cellules rouges du sang, ou (c) la liaison des cellules cytotoxiques du système immunitaire à des cellules tumorales, ou (d) la liaison des cytokines activantes, les substances cytotoxiques ou une protéase vers une cellule cible.

23. Kit de diagnostique renfermant un anticorps multimère Fv selon l'une des revendications 1 à 14.
